(19) Europäisches Patentamt
European Patent Office
Office européen des brevets

(11) **EP 4 260 889 A1**

(12) **EUROPEAN PATENT APPLICATION**

(43) Date of publication:
**18.10.2023 Bulletin 2023/42**

(21) Application number: **22305535.1**

(22) Date of filing: **13.04.2022**

(51) International Patent Classification (IPC):
*A61M 5/20* (2006.01)     *A61M 5/145* (2006.01)
*A61M 5/155* (2006.01)     *A61M 5/48* (2006.01)
*A61M 5/31* (2006.01)

(52) Cooperative Patent Classification (CPC):
**A61M 5/2033; A61M 5/14586; A61M 5/155;**
**A61M 5/2053; A61M 5/3135; A61M 5/484;**
A61M 2005/202; A61M 2005/3114;
A61M 2005/3142; A61M 2005/3143;
A61M 2005/31506; A61M 2005/31508;
A61M 2005/3151; A61M 2205/0216;
A61M 2205/3334;                    (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Centre national de la recherche scientifique**
**75016 Paris (FR)**

• **Université de Haute Alsace**
**68200 Mulhouse (FR)**

(72) Inventor: **LUCHNIKOV, Valeriy**
**68057 Mulhouser Cedex 2 (FR)**

(74) Representative: **Lavoix**
**2, place d'Estienne d'Orves**
**75441 Paris Cedex 09 (FR)**

(54) **DEVICE FOR A CONTROLLED EJECTION OF A LIQUID SOLUTION**

(57)     The invention concerns a device (10) comprising:
- a barrel (12);
- an elastic system (14) separating the barrel into a solution chamber (36) and a depressurized chamber (38) ;
- a first opening (16) to the solution chamber;
- a piston (18) able to be moved inside the depressurized chamber from a rest configuration to a pulled configuration in order to suck said solution into the solution chamber;
- a second opening (20) communicating with the depressurized chamber via a flow restrictor (21), and
- a regulation system (22) able to close the second opening to make the depressurized chamber airtight when the piston is sucking the solution and able to open the second opening to release the elastic system and to eject the solution out of the solution chamber.

FIG.3

**(Cont. next page)**

(52) Cooperative Patent Classification (CPC): (Cont.)
    A61M 2205/582

**Description**

FIELD OF THE INVENTION

The present invention concerns a device for a controlled ejection of a liquid solution. In particular, the liquid solution is a medical solution or a food solution.
The invention also concerns a method of loading the liquid solution into such a device.

BACKGROUND OF THE INVENTION

Many medical solutions require a slow mode of administration, in order to avoid undesirable and/or acute effects and to obtain the best therapeutic effect.
Slow injection with the use of traditional syringes requires special training for doctors and nurses, but is still a tedious and time-consuming operation, especially if large amounts of medication are to be administered to a patient. In addition, the rate of administration can vary from patient to patient.
Technical solutions allowing this type of administration are available but they require an external energy supply (electrical or mechanical) or a permanent and constraining human intervention. Moreover, they do not meet all the technical and financial requirements for use by the medical profession.
In particular, syringe and infusion pumps are efficient but not completely appropriate for mobile medical interventions because of their energy dependency and their price makes them not affordable devices. On the other side, elastomeric pumps and drip sets with bag may be considered as more mobile solutions but imply additional devices to operate, which is not suitable for emergency medical interventions. For example, the document WO 2002/22189 discloses such an elastomeric pump.

SUMMARY OF THE INVENTION

One of the aims of the invention is to propose a device for injecting a liquid solution, as a medical solution for example, at a slow and controlled flowrate, while remaining cost efficient.
For this purpose, the invention relates to a device for a controlled ejection of a liquid solution comprising:

- a barrel defining an internal volume;
- an elastic system separating the internal volume into a solution chamber and a depressurized chamber;
- a first opening configured to allow the inlet and the outlet of said solution into the solution chamber;
- a piston able to be moved inside the depressurized chamber from a rest configuration to a pulled configuration in order to suck said solution into the solution chamber through the first opening by the deformation of the elastic system creating a vacuum into the solution chamber;
- a second opening communicating with the depressurized chamber via a flow restrictor, and
- a regulation system able to close the second opening to make the depressurized chamber airtight when the piston is sucking the solution and able to open the second opening to allow an airflow to flow inside the depressurized chamber via the flow restrictor to release the elastic system and to eject the solution out of the solution chamber through the first opening.

Thanks to these features, the elastic system and the piston enable the suction of the solution inside the device through vacuum suction. The flow restrictor enables to control the airflow inlet inside the device and in consequence the flow rate of the solution ejection out of the device. The invention makes the work of the user, and notably a medical staff, more comfortable and less stressful, and limits the rate of administration to a safe level, without the need for expensive and complex electronic devices.
The device according to the invention comprises one or more of the following features, taken solely, or according to any technical feasible combination:

- the elastic system is an elastic membrane fixed relatively to the barrel, the elastic membrane being advantageously made of polyisoprene, silicone or latex;
- the elastic system is a mobile shutter in the internal volume of the barrel, the mobile shutter being connected to a spring arranged inside the depressurized chamber or inside the solution chamber;
- the device comprises a cap removably cooperating with the barrel and defining the first opening;
- the device comprises position-holding means configured to keep the piston in the pulled configuration;
- the position-holding means are a stop protruding from the barrel inside the internal volume, the stop being able to cooperate with positioners to maintain the piston in the pulled configuration;

- the device comprises a handle for moving the piston from the rest configuration to the pulled configuration;
- the regulation system comprises a screw able to move in a matching hole from a screwed-in configuration blocking the fluidic communication between the second opening and the flow restrictor, to an unscrewed configuration enabling the fluidic communication between the second opening and the flow restrictor;
- the flow restrictor is a capillary system extending between the second opening and the depressurized chamber;
- the capillary system comprises at least one capillary channel extending between the second opening and the depressurized chamber;
- the screw comprises externals threads and the matching hole comprises internal threads, the capillary system being a spiral capillary channel defined between the external threads and the internal threads.
- the flow restrictor is a porous track-etched membrane;
- the piston is able to move from the rest configuration to the pulled configuration through a translation movement, without rotation; and
- the device comprises a screw plunger cooperating with the piston, the piston being able to move from the rest configuration to the pulled configuration through a translation and rotation movement.

The invention also concerns a method of loading a liquid solution into a device the method comprising at least the following steps:

- providing a reservoir containing the solution,
- providing the device as defined above, the piston being in the rest configuration and the regulation system closing the second opening,
- placing the first opening in fluidic contact with solution contained in the reservoir,
- moving the piston from the rest configuration to the pulled configuration and sucking said solution into the solution chamber.

The invention also concerns a method of delivering a liquid solution loaded into such a device, the method comprising at least the following steps:

- providing the device as defined above, the device being loaded with said solution in the solution chamber, the piston being in the pulled configuration and the regulation system closing the second opening,
- operating the regulation system to open the second opening allowing an airflow to flow inside the depressurized chamber via the flow restrictor to release the elastic system,
- ejection of the solution out of the solution chamber through the first opening.

BRIEF DESCRIPTION OF THE DRAWINGS

The invention and its advantages will be better understood upon reading the following description, which is given solely by way of non-limiting example and which is made with reference to the appended drawings, in which:

- Figure 1 is a cross sectional view of a first embodiment of a device according to the invention in an assembled configuration;

- Figure 2 is a cross sectional view of the device of Figure 1 in a disassembled configuration;

- Figure 3 is a cross sectional view of the device of Figure 1 during the loading of the liquid solution;

- Figure 4 is a cross sectional view of the device of Figure 1 during the delivering of the liquid solution;

- Figure 5 is a cross sectional view of a second embodiment of a device according to the invention;

- Figure 6 is a cross sectional view of the device of Figure 5 during the delivering of the liquid solution;

- Figure 7 is a cross sectional view of a third embodiment of a device according to the invention,

- Figure 8 is a cross sectional view of a fourth embodiment of a device according to the invention,

- Figure 9 is a cross sectional view of a fifth embodiment of a device according to the invention, and

- Figure 10 is a cross sectional view of a sixth embodiment of a device according to the invention.

DESCRIPTION OF A FIRST EMBODIMENT OF THE INVENTION

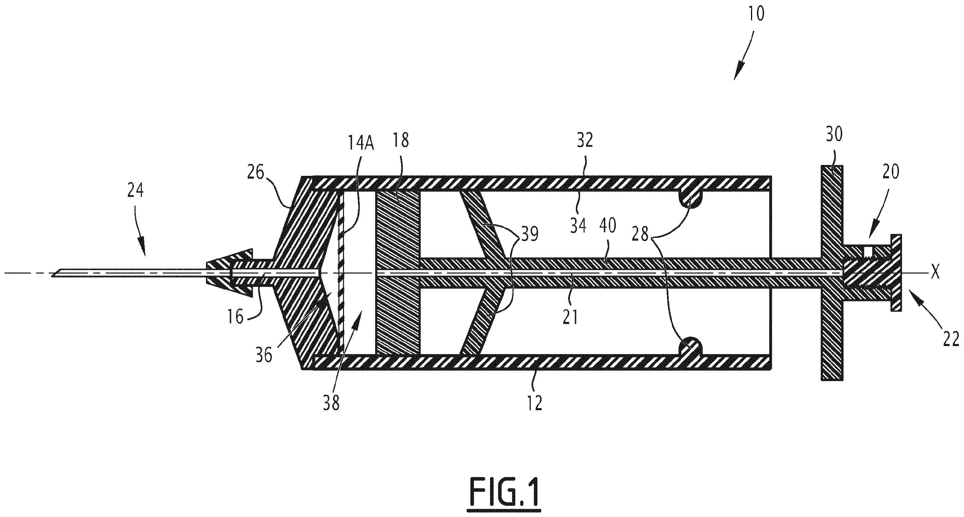

A device 10 for a controlled ejection of a liquid solution is represented on Figure 1.

The liquid solution is a liquid fluid, which may comprise solid particles floating in the fluid.

The liquid solution is notably a medical solution, in particular a medical solution with slow administration rates.

For example, intravenous injections of Tramamdol should be given slowly over 2 to 3 minutes.

As another example, each dose of Digoxin should be given by intravenous infusion over 10 to 20 minutes. Rapid intravenous injection may cause vasoconstriction producing hypertension and/or decreased coronary flow. A slow injection rate is therefore important in hypertensive heart failure and acute myocardial infarction.

As another example, adrenaline injection is the drug of choice for the first-line treatment of cardiopulmonary arrest, anaphylactic shock, and certain severe shock states (in resuscitation). For example, in case of a cardiac arrest, an intravenous injection of 0.5 mg during 5 minutes is carried out.

The device may be used for medicine administration by injection route for emergency care or humanitarian applications, for wildlife veterinarian applications and/or for the patient transportation in isolated environment.

The injection with the device may be intravenous, intramuscular, nasogastric or subcutaneous.

In a variant, the liquid solution may be a food solution. In particular, liquid nutrients should be delivered slowly into the gastrointestinal tract, for example at a rate lower than 30 ml/min, to avoid adverse effects such as abdominal discomfort, nausea, satiety and cramping.

In a variant, the liquid solution may be a glue, a silicone or a painting.

Referring to Figure 1, the device 10 extends along a longitudinal axis X.

The device 10 comprises a barrel 12, an elastic system 14, a first opening 16, a piston 18, a second opening 20, an air flow restrictor 21 and a regulation system 22.

The device 10 may further comprise a needle 24, a cap 26, position-holding means 28 and a handle 30.

The barrel 12 presents a cylindrical shape, extending along the longitudinal axis X. The transversal section of the barrel 12 is notably circular. In a variant, the transversal section may be a square or a hexagonal for example. The barrel 12 extends advantageously along a length comprised between 5 cm and 50 cm along the longitudinal axis X. The barrel 12 defines an external surface 32 and an internal surface 34. The barrel 12 defines an internal volume.

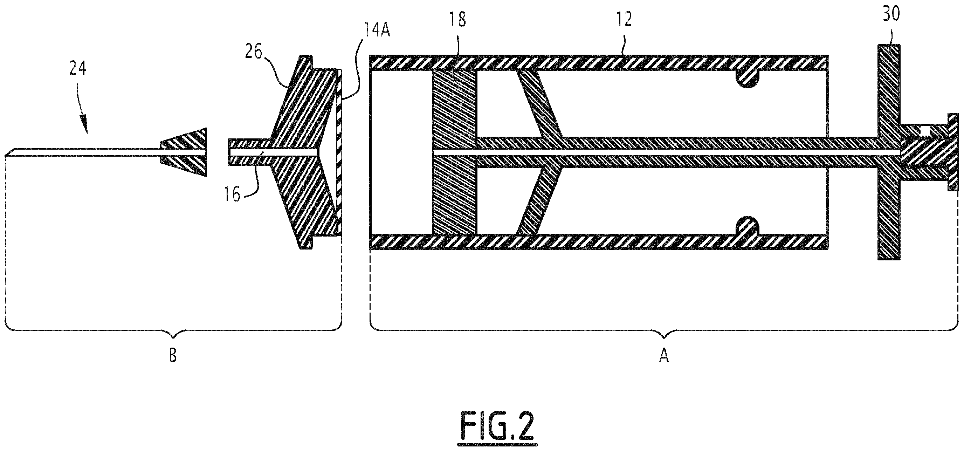

The cap 26 is arranged at an extremity of the barrel 12 along the longitudinal axis X. The cap 26 is advantageously made of plastic, for example polypropylene. The cap 26 is removably cooperating with the barrel 12. In other words, the cap 26 may be fixed to the barrel 12 in an assembled configuration of the device 10. The cap 26 may be easily removed from the barrel 12 by a user in a dissembled configuration of the device 10, represented on Figure 2.

Referring to Figure 2, the device 10 comprises a reusable part A composed notably of the barrel and the piston, and a disposable part B composed of the membrane 14A, the cap 26 and the needle 24. This enables to decrease the price of the medical device considering multiple uses. Moreover, the healthcare and the sterilization are carried out easily.

The cap 26 defines the first opening 16. The first opening 16 is notably defined at the center of the cap 26. In an alternative, the first opening 16 is off-center. The first opening 16 is a channel extending along the longitudinal axis X in the cap 26.

The elastic system 14 is here an elastic membrane 14A fixed relatively to the barrel 12. The elastic membrane 14A may be fixed to the internal surface 34 of the barrel 12. In a variant, the elastic membrane 14A is fixed to the cap 26, as represented on Figure 2, and when the device is assembled the elastic membrane 14A is thus fixed relatively to the barrel 12. The elastic membrane 14A is advantageously made of polyisoprene, silicone or latex.

As visible on the Figures, the elastic system 14 separates the internal volume into a solution chamber 36 and a depressurized chamber 38. In other words, there is no fluidic communication between the solution chamber 36 and the depressurized chamber 38, due to the elastic system 14.

The first opening 16 is configured to allow the inlet and the outlet of the solution into the solution chamber 36.

The needle 24 is arranged in the extension of the first opening 16. The needle 24 is fixed to the cap 26. In alternative, the device 10 is devoid of a needle.

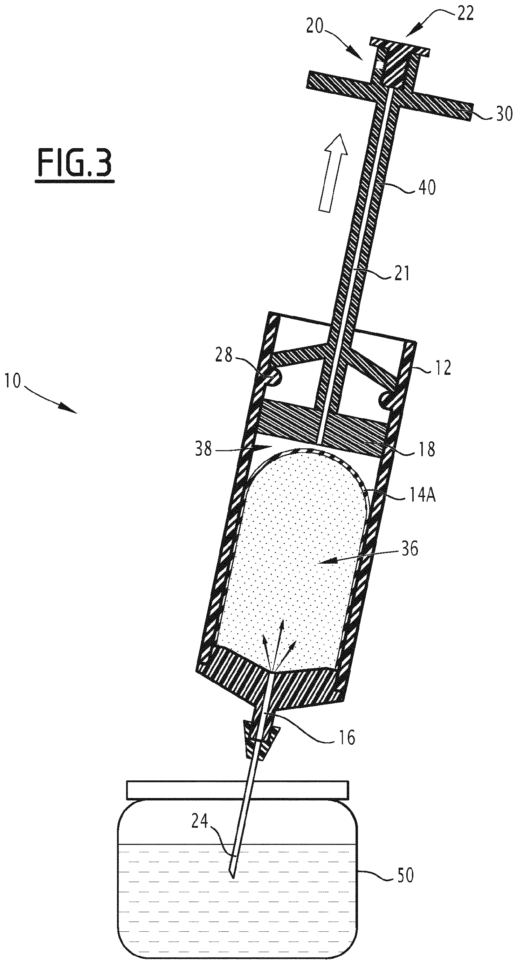

The piston 18 is able to be moved inside the depressurized chamber 38, by translation along the longitudinal axis X. Here, the piston 18 is able to move through a translation movement along the longitudinal axis X, without rotation. However, the skilled person will understand that the user may move the piston 18 through a translation movement with eventually a rotation movement. The piston 18 is able to move from a rest configuration, represented on Figure 1, to a pulled configuration, represented on Figure 3. In particular, when moving from the rest configuration to the pulled configuration, the piston 18 is moving in the opposite direction from the first opening 16. When moving from the rest configuration to the pulled configuration, the elastic membrane 14A is deformed. Indeed, the volume of the depressurized chamber 38 between the elastic membrane 14A and the piston 18 is airtight and the elastic membrane 14A therefore follows the movement of the piston 18 to maintain the balance between the atmospheric pressure, the pressure in the depressurized chamber 38and the tension of the elastic membrane 14A. This deformation of the elastic membrane 14A creates a vacuum into the solution chamber 36. In consequence, this vacuum sucks the solution from the outside of the device 10 into the solution chamber 36 part through the first opening 16, as illustrated on Figure 3.

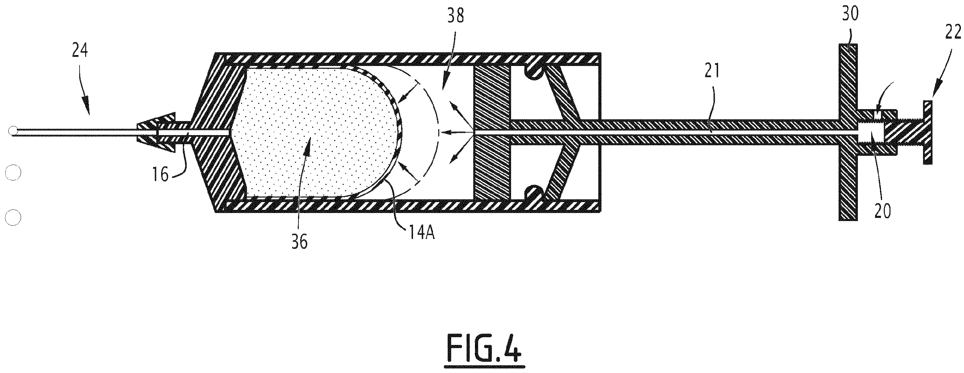

The position-holding means 28 are configured to keep the piston 18 in the pulled configuration. As visible in the Figure 1, the position-holding means 28 are here a stop protruding from the internal surface 34 of the barrel 12 inside the internal volume. The stop is in particular protruding circumferentially. The stop is able to cooperate with the piston 18, and in particular with positioners 39 to maintain the piston 18 in the pulled configuration, as visible on Figures 3 and 4.

The handle 30 is configured to move the piston 18 from the rest configuration to the pulled configuration. The handle 30 is connected to the piston 18 through a rod 40 extending along the longitudinal axis X. In particular, the handle 30 is designed to be grabbed by the hand of a user. By pulling the handle 30, the piston18 is pulled from the rest configuration to the pulled configuration

The second opening 20 communicates with the depressurized chamber 38 via the air flow restrictor 21. The flow restrictor 21 is here a capillary system extending between the second opening 20 and the depressurized chamber 38. The capillary system comprises at least one capillary channel extending along the longitudinal axis X between the second opening 20 and the depressurized chamber 38. The capillary channel(s) extend(s) inside the rod 40 and the piston 18. On the example represented on Figure 1, the capillary system comprises here a single capillary channel. In a variant, not shown, the capillary system comprises advantageously several capillary channels extending in parallel. Each capillary channel is thin enough to allow capillary action to take place. In particular, each capillary channel presents advantageously a diameter comprised between 0.05 mm to 1 mm.

As represented on Figure 3, the regulation system 22 is able to close the second opening 20 to make the vacuum chamber 28 airtight when the piston 18 is drawing the solution. As represented on Figure 4, the regulation system 22 is further able to open the second opening 20 to allow an airflow to flow inside the vacuum chamber 28 via the air flow restrictor 21 to release the elastic membrane 14A and to eject the solution out of the solution chamber 36 through the first opening 16.

The regulation system 22 comprises here a screw able to move in a matching hole from a screwed-in configuration blocking the fluidic communication between the second opening 20 and the flow restrictor 21, as represented on Figures 1 to 3, to an unscrewed configuration enabling the fluidic communication between the second opening 20 and the flow restrictor 21, as represented on Figure 4.

The solution flowrate out of the first opening 16 is linked to the flowrate of the air entering the depressurized chamber 38 through the flow restrictor 21. This air flowrate $Q_{AIR}$ is defined by the aerodynamic resistance in the capillary channel given by the Hagen-Poiseuille equation:

$$Q_{AIR} = (\pi r^4 / 8 \eta l) \Delta P$$

with viscosity $\eta$, capillary channel length $l$, capillary channel radius $r$, and pressure difference between the atmospheric pressure and the pressure of the depressurized chamber 38 $\Delta P = P_{ATM} - P_{VAC.CHAMB}$.

The high influence of $r^4$ term of aerodynamic resistance on capillary allows the control of the administration flowrate within a wide range of possibilities. Moreover, the skilled person will understand that by using N capillaries with different geometrical characteristics (length l, radius r), and thus respectively different aerodynamic resistance, the device 10 may deliver the liquid solution within a range of $2^N$ configurations.

OPERATION OF THE INVENTION

The operation of the device 10 will now be described.

The operation comprises a method of loading a liquid solution into the device 10, in reference to Figure 3.

A reservoir 50 containing the solution is provided. A device 10 as described above is also provided, the piston 18 being in the rest configuration and the regulation system 22 closing the second opening 20, as represented on Figure 1.

The first opening is placed in fluidic contact with the solution contained in the reservoir 50. In particular, the needle 24 is placed inside the reservoir 50. In the alternative in which the device 10 is devoid of a needle, the cap 26 and the first opening 16 are directly placed in contact with the solution contained in the reservoir 50.

Then, the user grabs the handle 30 and pulls it along the longitudinal axis X, as illustrated by the arrow represented on Figure 3. The translation movement of the handle 30 leads to the piston 18 to move from the rest configuration to the pulled configuration.

A depressurization is created in the depressurized chamber 38 whose pressure becomes lower than the atmospheric pressure. The elastomeric membrane 14A then expands because of this pressure difference, and the solution is sucked through the needle 24 and the first opening 16 to the solution chamber 36.

When the piston 18 is pulled enough to be locked in position in the barrel 12 thanks to the contact between the position-holding means 28 and the positioners 39, the pulled configuration is maintained due to the suction from the depressurized chamber 38 on the elastic membrane 14A which is now maintained in pulled configuration even if its elastic forces tend it to come back in rest configuration.

In alternative to the method of loading a liquid solution into the device 10 carried out by the user, the device 10 may be loaded upstream, for example in a factory, and sold directly loaded and in the pulled configuration to the user.

Then, the operation comprises a method of delivering a liquid solution, in reference to Figure 4.

The device 10 is connected to the patient body through the needle 24 directly inserted to the patient body, or alternatively using a medical tubing between the device 10 and the needle 24 or between the device 10 and a catheter.

As visible on Figure 4, the regulation system 22 is operated to open the second opening 20 allowing an airflow to flow inside the depressurized chamber 38 via the flow restrictor to release the elastic membrane 14A.

In particular, the screw is unscrewed, allowing the air to flow through the second opening 20, through the flow restrictor 21 and finally into the depressurized chamber 38 which implies the balance of the atmospheric pressure, the pressure exerted by the elastic membrane 14A on the solution, and the pressure in the depressurized chamber 38. It implies also the deflation of the solution chamber 36 under mechanical forces applied by the elastic membrane 14A on the solution.

In consequence, the solution is then pushed outside of the device 10 through the first opening 16 and the needle 24, and delivered for example to the patient.

The deliverance of the solution may be stopped at any time by closing the regulation system 22.

DESCRIPTION OF A SECOND EMBODIMENT OF THE INVENTION

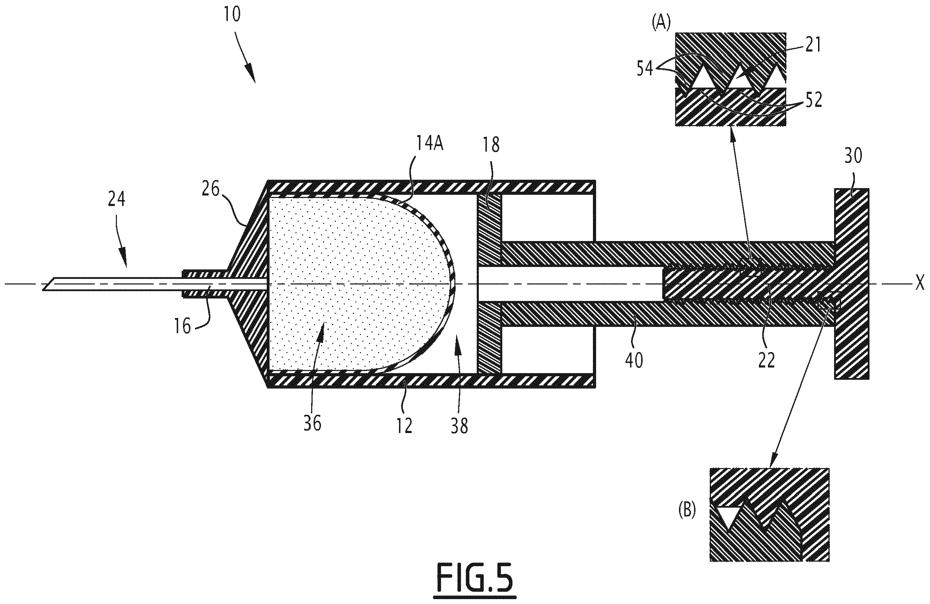

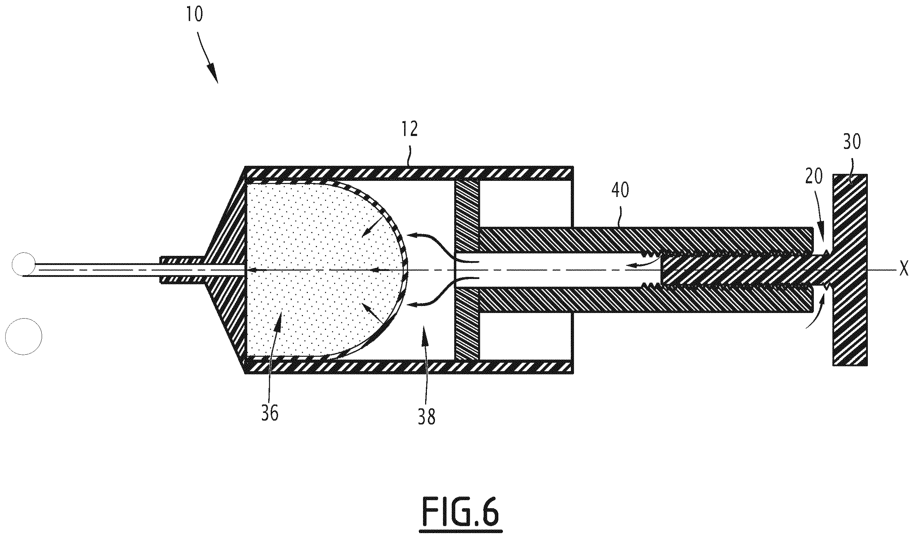

A second embodiment of the device 10 according to the invention will be described below, referring to Figures 5 and 6.

The device 10 according to the second embodiment is similar to device 10 according to the first embodiment explained above except the features described below.

In particular, the regulation system 22 comprises here a screw cooperating with a matching hole arranged in the rod 40. The screw comprises externals threads 52 and the matching hole comprises internal threads 54, as visible on the enlargement (A) of Figure 5.

As visible on Figures 5 and 6, the capillary system 21 is here a spiral capillary channel defined between the external threads 52 and the internal threads 54. The internal threads 54 are triangular. The external threads 52 are trapezoidal except the last threads arranged next to the handle 30, which are triangular, as visible on the enlargement (B).

In a variant, the external and the internal threads 52, 54 may be rectangular, so that the air capillary has a rectangular cross-section.

The regulation system 22 comprises here the screw able to move in the matching hole from a screwed-in configuration blocking the end of the spiral capillary channel, as visible on the enlargement (B) of Figure 5, to an unscrewed configuration opening the spiral capillary channel and enabling the air communication between the second opening 20 and the flow restrictor 21, as represented on Figure 6.

The piston 18, the rod 40 and the screw may be manufactured using standard methods of making internal and external threads.

The regulation system 22 enables to adjust the delivery rate of the solution. Indeed, as the aerodynamic resistance of the capillary is proportional to its length, the delivery rate may be adjusted within by the number of screw turns.

In particular, in case of the rectangular threads, the formula for the aerodynamic resistance of the spiral capillary channel reads as follows:

$$R \approx \frac{12}{1 - 0.63(h/w)} \frac{\eta\sqrt{(2\pi r)^2 + a^2}}{h^3 w}(N - n)$$

The flow rate Q of the air in the device 10 is :

$$Q = \frac{\Delta P}{R} = \frac{1 - 0.63(h/w)}{12} \frac{h^3 w}{\eta\sqrt{(2\pi r)^2 + a^2}} \frac{\Delta P}{N - n}$$

where h,w are the dimensions of the cross-sectional area, $\eta$ is the viscosity of the air, L is the length of the capillary, N is the number of turns required to completely separate the screw from the device, n is the number of turns made to open the capillary.

As visible on the Figures 5 and 6, the device 10 according to the second embodiment is devoid of position-holding means 28. The friction coefficient between the piston 18 and the barrel 12 is here sufficiently high to maintain the piston 18 in position when the solution chamber 56 is loaded, and the elastic membrane is inflated.

The operation of the second embodiment is similar to the operation of the first embodiment and will not be described again.

## DESCRIPTION OF A THRID EMBODIMENT OF THE INVENTION

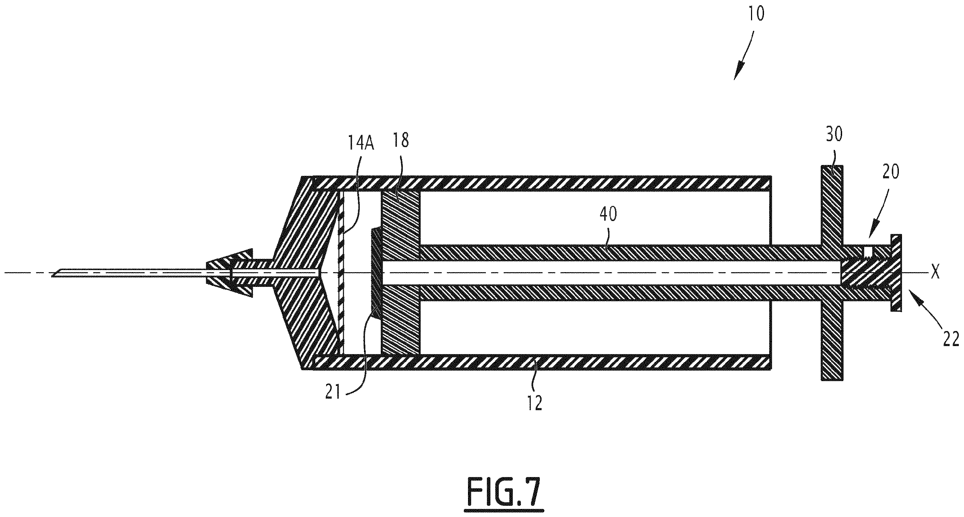

A third embodiment of the device 10 according to the invention will be described below, referring to Figure 7.

The device 10 according to the third embodiment is similar to device according to the first embodiment explained above except the features described below.

The air flow restrictor 21 is here a porous track-etched membrane. A track-etched membrane is a porous system comprising a thin polymer foil with channels crossing the membrane.

The porous track-etched membrane is arranged in the depressurized chamber 38 and covers the extremity of the channel arranged inside the rod 40 between the depressurized chamber 38 and the second opening 20.

The porous track-etched membrane is produced by irradiation of a polymer membrane by ions, accelerated to the energy of a few MeV in an industrial particle accelerator, followed by chemical etch if the ion tracks in the polymer. A broad spectrum of porous track-etched membranes exists with different permeabilities to air. For example, a 25 $\mu$m thick polycarbonate films, with a pore size of 0.05 $\mu$m, a porosity of 1.2%, is associated to an air flow rate of the order of 20 mL.min$^{-1}$.cm$^{-2}$ at a pressure difference of 0.7 bar, while at the pore size of 0.05 $\mu$m and a porosity 4.7% and with a same pressure difference, the air flow rate increases to approximatively 200 mL.min$^{-1}$.cm$^{-2}$.

As visible on the Figure 7, the device 10 according to the third embodiment is devoid of position-holding means 28, in a similar way to the second embodiment.

The operation of the third embodiment is similar to the operation of the first embodiment and will not be described again.

## DESCRIPTION OF A FOURTH EMBODIMENT OF THE INVENTION

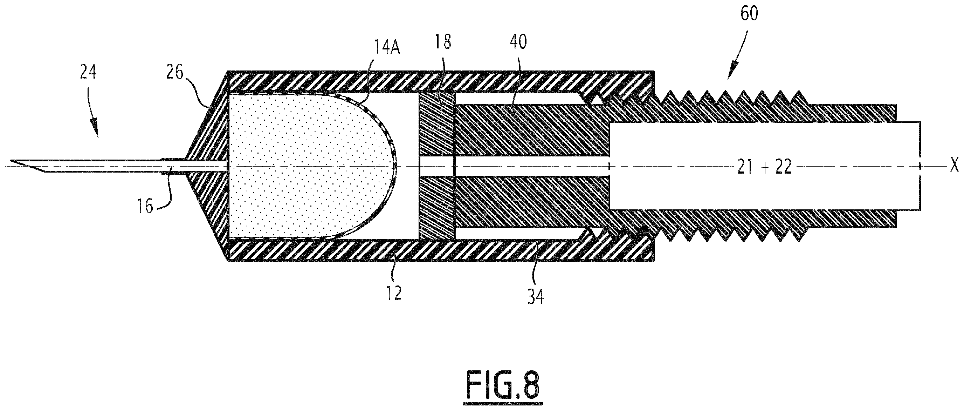

A fourth embodiment of the device 10 according to the invention will be described below, referring to Figure 8.

The device 10 according to the fourth embodiment is similar to device according to the first embodiment explained above except the features described below.

The device 10 comprises a screw plunger 60 configured to move the piston 18 along the longitudinal axis X.

To that effect, the screw plunger 60 is cooperating with the piston 18, through the rod 40. The rod 40 comprises external threads cooperating with internal threads arranged on the internal surface 34 of the barrel 12.

By rotation the screw plunger 60, the screw plunger 60 is able to move the piston 18 from the rest configuration to the pulled configuration. Here, the piston 18 is able to move from the rest configuration to the pulled configuration through a translation movement and a rotation movement along the longitudinal axis X.

The screw plunger 60 acts as position-holding means.

The flow restrictor 21 and the regulation system 22 are shown on Figure 8 schematically by a box. The flow restrictor 21 and the regulation system 22 may be implemented as in the first embodiment, second embodiment or third embodiment.

The operation of the fourth embodiment is similar to the operation of the first embodiment and will not be described again.

## DESCRIPTION OF A FIFTH EMBODIMENT OF THE INVENTION

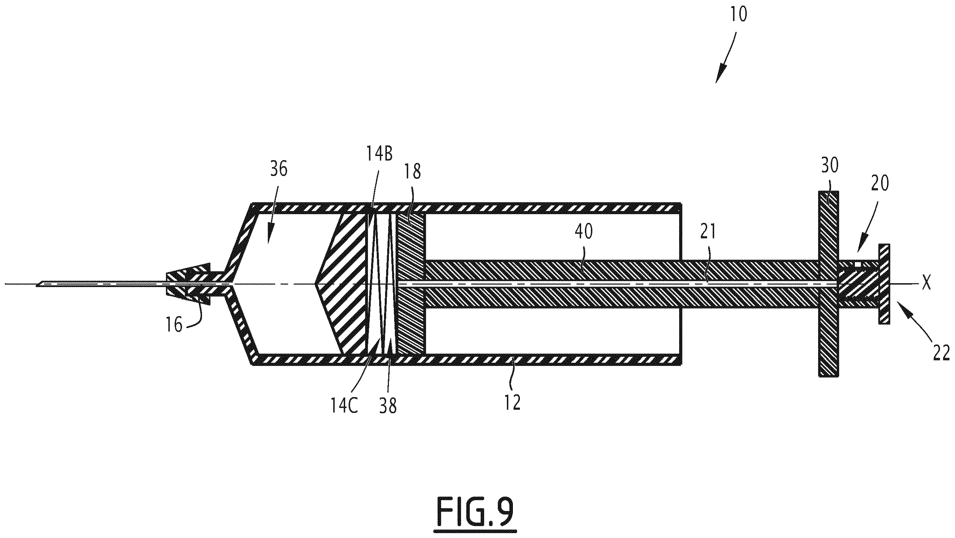

A fifth embodiment of the device 10 according to the invention will be described below, referring to Figure 9.

The device 10 according to the fifth embodiment is similar to device according to the first embodiment explained above except the features described below.

The elastic system 14 is here a mobile shutter 14B in the internal volume of the barrel 12. The mobile shutter 14B is in particular able to move along the longitudinal axis X between the first opening 16 and the piston 18.

As visible on Figure 9, the mobile shutter 14B is connected to a spring 14C arranged inside the depressurized chamber 38 between the mobile shutter 14B and the piston 18.

The spring 14C may be a helical spring. In a variant, the spring 14C may be a conical spring allowing a smaller footprint in the depressurized chamber 38.

When moving from the rest configuration to the pulled configuration, the length of the spring is hold equal as the volume of the depressurized chamber 38 between the mobile shutter 14B and the piston 18 is airtight. The mobile shutter 14B therefore follows the movement of the piston 18 to maintain a constant internal volume in the depressurized chamber 38. This movement of the mobile shutter 14B creates a vacuum into the solution chamber 36. In consequence, this vacuum sucks the solution from the outside of the device 10 into the solution chamber 36 part through the first opening 16, as illustrated on Figure 9.

When the regulating system 22 opens the second opening 20, air flows in the depressurized chamber 38, allowing the spring to relax to its extended state and to push the mobile shutter 14B in the direction of the first opening 16. The shutter pushes then the solution outside the device.

As visible on the Figure 9, the device 10 according to the fifth embodiment is devoid of position-holding means 28, in a similar way to the second embodiment.

## DESCRIPTION OF A SIXTH EMBODIMENT OF THE INVENTION

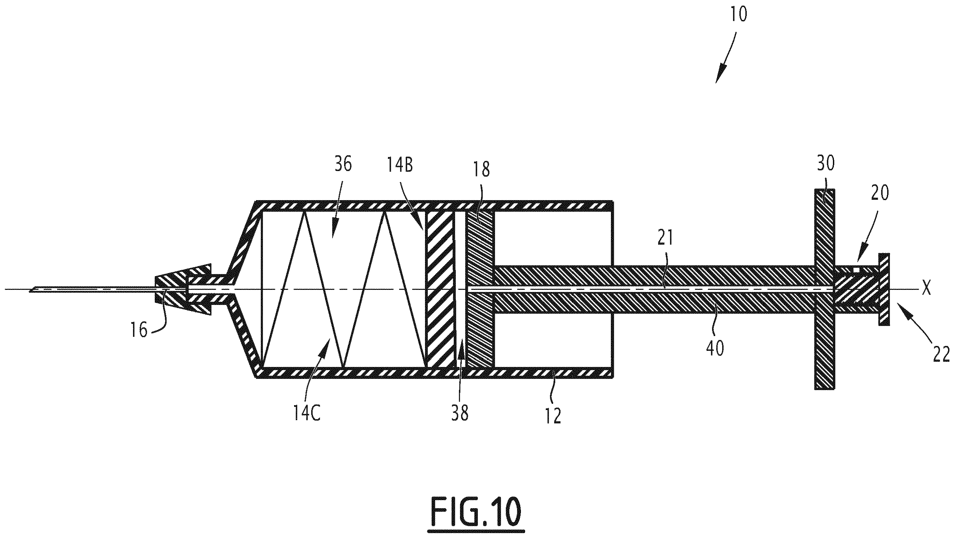

A sixth embodiment of the device 10 according to the invention will be described below, referring to Figure 10.

As in the fifth embodiment, the elastic system 14 is a mobile shutter 14B in the internal volume of the barrel 12. The mobile shutter 14B is in particular able to move along the longitudinal axis X between the first opening 16 and the piston 18.

As visible on Figure 10, the mobile shutter 14B is connected to a spring 14C arranged inside the solution chamber 36 between the mobile shutter 14B and the cap 26.

The spring 14C may be a helical spring. In a variant, the spring 14C may be a conical spring allowing a smaller footprint in the depressurized chamber 38.

When moving from the rest configuration to the pulled configuration, vacuum is created in the depressurized chamber 38 between the piston 18 and the shutter 14B. The mobile shutter 14B therefore follows the movement of the piston 18 to maintain a constant internal pressure in the depressurized chamber 38. This movement of the mobile shutter 14B creates a vacuum into the solution chamber 36. In consequence, this vacuum sucks the solution from the outside of the device 10 into the solution chamber 36 part through the first opening 16, as illustrated on Figure 10.

When the regulating system 22 opens the second opening 20, air flows in the depressurized chamber 38, allowing the spring 14C to relax to its unstrained state and to push the mobile shutter 14B in the direction of the first opening 16. The shutter 14B pushes then the solution outside the device.

As visible on the Figure 10, the device 10 according to the sixth embodiment is devoid of position-holding means 28, in a similar way to the second embodiment.

## DESCRIPTION OF OTHER EMBODIMENTS OF THE INVENTION

It will be apparent to those skilled in the art that other embodiments may be carried out in various ways by combining the previous embodiments.

For example, the first embodiment of the device 10 may be implemented without position-holding means 28 and/or positioners 39.

For example, the device 10 according to the third, fifth or the sixth embodiment may be implemented with a position-holding means 28 and/or positioners 39. For example, the flow restrictor 21 and the regulation system 22 may be implemented as in the third embodiment.

As another example, the fifth embodiment of the device 10 may be implemented with the regulation system 22 described in the third embodiment.

**Claims**

1.  A device (10) for a controlled ejection of a liquid solution comprising:

    - a barrel (12) defining an internal volume;
    - an elastic system (14) separating the internal volume into a solution chamber (36) and a depressurized chamber (38) ;
    - a first opening (16) configured to allow the inlet and the outlet of said solution into the solution chamber (36);
    - a piston (18) able to be moved inside the depressurized chamber (38) from a rest configuration to a pulled configuration in order to suck said solution into the solution chamber (36) through the first opening (16) by the deformation of the elastic system (14) creating a vacuum into the solution chamber (36);
    - a second opening (20) communicating with the depressurized chamber (38) via a flow restrictor (21), and
    - a regulation system (22) able to close the second opening (20) to make the depressurized chamber (38) airtight when the piston (18) is sucking the solution and able to open the second opening (20) to allow an airflow to flow inside the depressurized chamber (38) via the flow restrictor (21) to release the elastic system (14) and to eject the solution out of the solution chamber (36) through the first opening (16).

2. The device (10) according to claim 1, wherein the elastic system (14) is an elastic membrane (14A) fixed relatively to the barrel (12), the elastic membrane (14A) being advantageously made of polyisoprene, silicone or latex.

3. The device (10) according to claim 1, wherein the elastic system (14) is a mobile shutter (14B) in the internal volume of the barrel (12), the mobile shutter (14B) being connected to a spring (14C) arranged inside the depressurized chamber (38) or inside the solution chamber (36).

4. The device (10) according to any of the preceding claims, wherein the device (10) comprises a cap (26) removably cooperating with the barrel (12) and defining the first opening (16).

5. The device (10) according to any of the preceding claims, wherein the device (10) comprises position-holding means (28) configured to keep the piston (18) in the pulled configuration.

6. The device (10) according to claim 5, wherein the position-holding means (28) are a stop protruding from the barrel (12) inside the internal volume, the stop being able to cooperate with positioners (39) to maintain the piston (18) in the pulled configuration.

7. The device (10) according to any of the preceding claims, wherein the device (10) comprises a handle (30) for moving the piston (18) from the rest configuration to the pulled configuration.

8. The device (10) according to any of the preceding claims, wherein the regulation system (22) comprises a screw able to move in a matching hole from a screwed-in configuration blocking the fluidic communication between the second opening (20) and the flow restrictor (21), to an unscrewed configuration enabling the fluidic communication between the second opening (20) and the flow restrictor (21).

9. The device (10) according to any of the preceding claims, wherein the flow restrictor (21) is a capillary system extending between the second opening (20) and the depressurized chamber (38).

10. The device (10) according to claim 9, wherein the capillary system comprises at least one capillary channel extending between the second opening (20) and the depressurized chamber (38).

11. The device (10) according to claims 8 and 9, wherein the screw comprises externals threads (52) and the matching hole comprises internal threads (54), the capillary system being a spiral capillary channel defined between the external threads (52) and the internal threads (54).

12. The device (10) according to any of the claims 1 to 8, wherein the flow restrictor (21) is a porous track-etched membrane.

13. The device (10) according to any of the preceding claims, wherein the piston (18) is able to move from the rest configuration to the pulled configuration through a translation movement, without rotation.

14. The device (10) according to any of the claims 1 to 12, wherein the device (10) comprises a screw plunger (60) cooperating with the piston (18), the piston (18) being able to move from the rest configuration to the pulled configuration through a translation and rotation movement.

15. A method of loading a liquid solution into a device (10), the method comprising at least the following steps:

- providing a reservoir (50) containing the solution,
- providing the device (10) according any of the preceding claims, the piston (18) being in the rest configuration and the regulation system (22) closing the second opening,
- placing the first opening (16) in fluidic contact with solution contained in the reservoir (50),
- moving the piston (18) from the rest configuration to the pulled configuration and sucking said solution into the solution chamber (56).

FIG.1

FIG.2

# FIG.3

FIG.4

EP 4 260 889 A1

FIG.5

FIG.6

FIG.7

EP 4 260 889 A1

FIG.8

FIG.9

FIG.10

Europäisches
Patentamt

European
Patent Office

Office européen
des brevets

# EUROPEAN SEARCH REPORT

Application Number

EP 22 30 5535

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 176 642 A (CLEMENT THOMAS P [US]) 5 January 1993 (1993-01-05) * column 4, line 19 - column 7, line 46; figures 1-3 * ----- | 1-15 | INV. A61M5/20 A61M5/145 A61M5/155 A61M5/48 A61M5/31 |
| A | US 2015/343149 A1 (COWAN KEVIN P [US] ET AL) 3 December 2015 (2015-12-03) * paragraph [0020] - paragraph [0045]; figures 1a-1c,5a-5c * ----- | 1-15 | |
| A | DE 38 27 525 A1 (CIRBUS RUDOLF [DE]; GROZA IGOR [DE]) 22 February 1990 (1990-02-22) * column 2, line 32 - column 3, line 1; figures 4-7 * ----- | 1-15 | |

TECHNICAL FIELDS SEARCHED (IPC)

A61M

The present search report has been drawn up for all claims

| Place of search | Date of completion of the search | Examiner |
|---|---|---|
| Munich | 13 September 2022 | Walther, Manuel |

EPO FORM 1503 03.82 (P04C01)

## ANNEX TO THE EUROPEAN SEARCH REPORT
## ON EUROPEAN PATENT APPLICATION NO.

EP 22 30 5535

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2022

| Patent document cited in search report | | Publication date | Patent family member(s) | | Publication date |
|---|---|---|---|---|---|
| US 5176642 | A | 05-01-1993 | AU | 1765392 A | 06-10-1992 |
| | | | US | 5176642 A | 05-01-1993 |
| | | | WO | 9215350 A1 | 17-09-1992 |
| US 2015343149 | A1 | 03-12-2015 | EP | 2968761 A1 | 20-01-2016 |
| | | | HK | 1222348 A1 | 30-06-2017 |
| | | | JP | 2016515016 A | 26-05-2016 |
| | | | US | 2014276451 A1 | 18-09-2014 |
| | | | US | 2015343149 A1 | 03-12-2015 |
| | | | US | 2016030674 A1 | 04-02-2016 |
| | | | WO | 2014164685 A1 | 09-10-2014 |
| DE 3827525 | A1 | 22-02-1990 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200222189 A